Europäisches Patentamt

European Patent Office

Office européen des brevets

(11) Publication number: **0 322 429 B1**

# EUROPEAN PATENT SPECIFICATION

(45) Date of publication of patent specification: **19.10.94**  (51) Int. Cl.5: **C11D  3/386**

(21) Application number: **88905333.6**

(22) Date of filing: **31.05.88**

(86) International application number:
**PCT/US88/01844**

(87) International publication number:
**WO 88/09367 (01.12.88 88/26)**

The file contains technical information submitted
after the application was filed and not included in
this specification

(54) **CUTINASE CLEANING COMPOSITION.**

(30) Priority: **29.05.87 US 56500**

(43) Date of publication of application:
**05.07.89 Bulletin  89/27**

(45) Publication of the grant of the patent:
**19.10.94 Bulletin  94/42**

(84) Designated Contracting States:
**AT BE CH DE FR GB IT LI LU NL SE**

(56) References cited:
**EP-A- 0 268 452**

**"American Type Culture Collection", Receit
concerning ATCC Designation 53552**

(73) Proprietor: **GENENCOR INTERNATIONAL, INC.
4 Cambridge Place,
1870 South Winston Road
Rochester, New York 14618 (US)**

(72) Inventor: **KOLATTUKUDY, Pappachan
2301 Hoxton Court
Columbus, OH 43220 (US)**
Inventor: **POULOSE, Ayrookaran, J.
2540 Carmel Drive
San Bruno, CA 94066 (US)**

(74) Representative: **Armitage, Ian Michael et al
MEWBURN ELLIS
York House
23 Kingsway
London WC2B 6HP (GB)**

**Description**

Background of the Invention

a) Field of the Invention

The invention relates to enzymatic cleaning compositions and methods for using them. Particularly the invention relates to cleaning compositions comprising a surfactant and a cutinase enzyme.

b) Background Information

A wide variety of enzymes are well known for use in cleaning compositions. The use of B. subtilisins and B. licheniformis protease in commercial preparations is common. Other enzymes have also been used in commercial cleaning compositions such as, for examples, U.S. Patent No. 4,011,169, and British Patnet No. 1,293,613. Also a comprehensive review article of lipases in cleaning compositions can be found in Journal of Applied Biochemistry, 2:218-229 (1980) in an article entitles "Lipases as Detergent Components". Lipolytic detergent additives are also known from e.g., British Patent Specification No. 1,293,613 and Canadian Patent No. 835,343.

U.S. Patent No. 3,950,277 and British Patent Specification No. 1,442,418 disclose lipase enzymes combined with an activator and calcium and/or magnesium ions, respectively, which are utilized to pre-soak soiled fabrics and to remove triglyceride stains and soils from polyester or polyester/cotton fabric blends, respectively, Suitable microbial lipases for use therein (apart from animal and plant derived lipases) are said to those derived from Pseudomonas, Aspergillus, Pneumococcus, Staphylococcus, and Staphylococcus toxins, Mycobacterium tuberculosis, Mycotorula lipolytica, and Sclerotinia.

British Patent Specification No. 1,372,034 discloses a detergent composition comprising a bacterial lipase produced by Pseudomonas stutzeri strain ATCC 19154. Furthermore, it is recommended that the preferred lipolytic enzymes should have a pH optimum between 6 and 10, and should be active in said range, preferably between 7 and 9. Around 1970, this presumed Pseudomonas stutzeri strain was reclassified as Pseudomonas aeruginosa, as appears for example from the ATCC catalogues.

European Patent Application EP-A-0130064 discloses an enzymatic detergent additive comprising a lipase isolated from Fusarium oxysporum with an alleged higher lipolytic cleaning efficiency than conventional lipases.

In European Patent Application No. 0214761, Enzymatic detergent additives are described as the active component of which is a microbially produced lipase from a strain of Pseudomonas oepacia. The lipases described therein are claimed to be superior to the lipolytic detergent action of the prior art, especially at low temperature washing processes (around 60°C and below).

In PCT Patent Application No. 87/00859 other novel lipolytic enzymes are described as having an optimal pH in the range of 8 - 10.5 at a temperature of 60°C or less from bacterial strains selected from Pseudomonas pseudoalcaligenes, P. stutzeri and Acinetobacter calcoacetious. These enzymes are described as particularly effective at low temperatures; i.e., 40°C or lower and effective in both liquid and solid detergent compositions.

Also in U.S. Patent No. 3,950,277, it is described in general terms that lipases from Pseudomonas are suited as agents for removal of oily stains from fabrics, if used together with a special group of lipase activators. The art cited does not, however, cover cutinase enzymes from Pseudomonas or any other microbial source. However, prior art enzymes for use in cleaning compositions, while effective on many proteins and lipids, are not completely effective against all stains commonly found in laundry and other cleaning applications.

Further, many lipases are not stable at pH 8 - 11 where most cleaning compositions are used. Even further, most enzymes for use in cleaning compositions are not very stable, if at all, under oxidative conditions or in the presence of other enzymes such as proteases.

SUMMARY OF THE INVENTION

Accordingly it has been discovered that combinations of a surfactant and a substantially pure microbial cutinase enzyme are effective compositions for cleaning applications together with one or more enzymes selected from lipases, amylases and proteases. The cutinase enzyme preparations possess activity at pH of from about 8 and 11, exhibit cleaning activity in aqueous solution at concentrations from about .05 mg/l to about 100 mg/l or more at temperatures from about 20°C to about 50°.

The cutinase enzymes are oxidatively stable and stable in the presence of other enzymes such as proteases. Even further, the cutinases show a synergistic effect when a plurality of surfactants are used with the cutinase.

The invention also relates to the improved process for enzymatically cleaning a material with an aqueous solution; the improvement comprising adding a substantially pure cutinase and one or more enzymes selected from lipases, amylases and proteases to the cleaning solution.

DETAILED DESCRIPTION OF THE INVENTION

Applicant has discovered that cutinase enzymes are useful when included in cleaning compositions. These compositions may take on a variety of forms such as for laundry cleaning, household and industrial cleaning, and the like. The cleaning compositions comprise combinations of known surfactants and a microbial cutinase enzyme which can be used to clean a wide variety of materials. The composition can be added to aqueous solution or solid powder, or formulated in an aqueous solution or solid powder and used according to conventional cleaning techniques.

Enzyme

Cutinases are well known in the art and are available from a wide variety of sources. See Cutinases from Fungi and Pollen, P.E. Kolattukudy, pg. 472 - 504, incorporated herein by reference, for discussion of cutinases useful in the practice of the invention. A preferred cutinase is that cutinase isolated in a substantially pure form from Pseudomonas putida, particularly from the strain deposited as, ATCC 53552, described in copending U.S. patent application, Serial No. 932,959 filed November 19, 1986 (equivalent to EP-A-0 268 452), which enzyme therefrom has the following amino acid sequence:

```
                                  1                                    10
                                 ala pro leu pro asp thr pro gly ala pro

                                       20                              30
        phe pro ala val ala asn phe asp arg ser gly pro tyr thr thr ser ser gln ser glu

                                       40                              50
        gly pro ser cys arg ile tyr arg pro arg asp leu gly gln gly gly val arg his pro

                                       60                              70
        val ile leu trp gly asn gly thr gly ala gly pro ser thr tyr ala gly leu leu ser

                                       80                              90
        his trp ala ser his gly phe val val ala ala ala glu thr ser asn ala gly thr gly

                                      100                             110
        arg glu met leu ala cys leu asp tyr leu val arg glu asn asp thr pro tyr gly thr

                                      120                             130
        tyr ser gly lys leu asn thr gly arg val gly thr ser gly his ser gln gly gly gly

                                      140                             150
        gly ser ile met ala gly gln asp thr arg val arg thr thr ala pro ile gln pro tyr

                                      160                             170
        thr leu gly leu gly his asp ser ala ser gln arg arg gln gln gly pro met phe leu

                                      180                             190
        met ser gly gly gly asp thr ile ala phe pro tyr leu asn ala gln pro val tyr arg

                                      200                             210
        arg ala asn val pro val phe trp gly glu arg arg tyr val ser his phe glu pro val

                                      220                             230
        gly ser gly gly ala tyr arg gly pro ser thr ala trp phe arg phe gln leu met asp

                                      240                             250
        asp gln asp ala arg ala thr phe tyr gly ala gln cys ser leu cys thr ser leu leu

                                      258
        trp ser val glu arg arg gly leu
```

Other sources of bacterial and fungal cutinases include:
Fusarium solani pisi
Fusarium roseum sambucinum
Fusarium roseum culmorum
Helminthosporum sativum
Ulocladium consortiale
Streptomyces scabies
Colletotrichum capsici

4

Phytopthora cactorum
Botrytis cineria
Colletrotrichum gloesosporioides

The cutinase of the invention should preferably be selected to cause at least about 10%, and preferably 20%, hydrolysis of the given fat under given conditions. Normally the amount would be in a concentration of from about .01% to about 5.0% by weight of the surfactant, and preferably from about .05% to about 3%, such that upon dilution in wash water it is in a concentration of at least about .05mg/L. Further, one skilled in the art could take the preferred cutinase or, for that matter, any cutinase of the invention or any immunologically identical cutinase and use random or selective replacement of amino acids to produce other cutinases which are more or less selective toward given substrates or include modification in activity such as oxidative stability. Substantially pure cutinase includes the isolated enzyme as well as the broth containing the enzyme in unpurified form but essentially free of other enzyme and enzyme sources.

The natural substrate of cutinase is cutin which is a biopolyester polymer which covers the plant leaves, fruits, etc., see Structure, Biosyntheses and Biodegradatin of Cutin and Suberin. (1981), P.E. Kolattukudy, Ann. Rev. Plant Physiol., 32, pgs. 539-567. Stains comprising lipids which could be hydrolyzed or bound by cutinase on a substrate such as cloth would be similar to the natural substrate cutin. Cutinase, for these types of stains, will be more effective than the prior art lipases. The cutinases will work especially well on gravy, oils and greases, plant or grass, oil based makeup and collar stains.

Cutinases are distinguishable from other lipases by methods well known in the art, See R.E. Purdy and P.E. Kolattukudy, Biochemistry, "Cutinase Assay", 14:2831-2840, (1975). Microbial cutinases from both fungal and bacterial sources have very good activity at pH 8-pH 11 which is an ideal pH condition for detergent use.

Because of the specific activity of cutinases, one or more other cutinases, or one or more other enzymes, such as proteases, amylases or other lipases, are included along with the cutinase in the cleaning composition of the invention. Further, Applicant shows a synergistic increase in hydrolytic activity of cutinase when two or more surfactants are combined along with the cutinase enzyme.

Cutinases then are ideal for cleaning composition inclusion. They have stability oxidatively such as in $H_2O_2$. They have good stability in a temperature range of from about 20 - 50°C which is ideal from a cleaning point of view. They are also stable in the presence of other enzyme; e.g., proteases, and as such, are ideal for mixtures of enzymes.

The Surfactant

A number of known compounds are suitable surfactants useful in the present compositions. These include nonionic, anionic, cationic, or zwitterionic detergents, as disclosed in U.S. 4,404,128 to Barry J. Anderson and U.S. 4,261,868 to Jiri Hora et al. The art is familiar with the different formulations which can be used as cleaning compositions.

The Clearing Compositions and Method of Use

Cutinases can be formulated as a purposefully added ingredient into known powdered and liquid detergents having pH between 6.5 and 12.0 at levels of about 0.01 to about 5% (preferably .05 to .5%) by weight of the detergent. These detergent cleaning compositions also include one or more enzymes selected from proteases, lipases and amylases, and possibly also bleaches, colorants, builders, and stabilizers.

The cutinase of the invention may be added to powdered detergents in the form of granulates or prills, prepared by methods known in the art such as described in British Patent Nos. 1,324,116 and 1,362,365 and U.S. patent Nos. 3,519,570; 4,106,991 and 4,242,219.

The cutinase preparations of the invention can be prepared by cultivating the microorganisms defined herein or otherwise cutinase containing microorganism under appropriate conditions. In order to obtain reasonable yields of enzyme, media containing readily assimilable carbon and energy sources as necessary such as a nitrogen source, as well as calcium and magnesium salts and trace elements and cutin, or monomers of cutin, or compounds resembling cutin or cutin monomers. One could also obtain the gene for cutinan and express in any organism of choice where one may not have to add cutin or cutin monomers into the fermentation.

The addition of cutinase to conventional cleaning compositions does not create any special use limitation. In other words, any temperature and pH suitable for detergent compositions containing enzymes is also suitable for the present compositions.

5

Although the preferred form of the invention has been described above, it will be obvious to those skilled in the art to which the invention pertains, that, after understanding the invention and in view of the following testing as a whole, various changes and equivalent modifications may be made without parting from the scope of the invention as defined by the claims.

STABILITY CUTINASE AGAINST PROTEASES

Reaction conditions:

Buffer: 0.1 M NaP, pH 10
Temp: 37°C
Lipase: 42 ug/ml
Approximately 1:1 protease:cutinase aqueous solution were made up with the following results.

ENZYME ACTIVITY

| Protease Incubation Time | 0 min | 5 min | 10 min | 15 min | 14 hrs |
|---|---|---|---|---|---|
| None | 1.57 | 1.60 | 1.47 | 1.63 | 1.61 |
| Maxacal (35ug/ml) | 1.68 | 1.58 | 1.72 | 1.66 | 0.134 |
| Esperase (64 ug/ml) | 1.73 | 1.64 | 1.59 | 1.51 | 0.456 |

Maxical is Gist-Brocade's brand of subtilisin enzyme (protease) Esperase is Novo's brand of protease enzyme (protease)

TEMPERATURE STABILITY OF BACTERIAL CUTINASE

| HALF LIFE AT 50°C | |
|---|---|
| pH | Hrs. |
| 7 | 30 |
| 8 | 25 |
| 9 | 12 |
| 10 | 0.3 |

Enzyme was incubated at 50°C in 0.1M sodium phosphate buffer at various pH's and activity was measured by hydrolysis of trioctanoin in polyvinyl alcohol emulsions.

EFFECT OF DETERGENTS ON HYDROLASE ACTIVITY

Reaction Conditions:

substrate: p-nitro-phenyl butyrate, 1mm (pnp)
pH: 8.0
buffer: 0.1m tris pH 8.0
temperature: 25°C
enzyme: bacterial cutinase from ATCC 53552
An aqueous solution with the following were made up and the enzyme activity was measured in these solutions using pnp as a substrate by following absorbance of p-nitrophenol at 410 mm.

| Triton x100% | SDS % | % Activity |
|---|---|---|
| 0 | 0 | 100 |
| 0.2 | -- | 78 |
| 0.4 | -- | 60 |
| -- | 0.05 | 30 |
| -- | 0.1 | 23 |
| -- | 0.2 | 14 |
| -- | 0.4 | 6 |
| 0.4 | 0.4 | 78 |
| 0.2 | 0.2 | 98 |
| 0.2 | 0.05 | 125 |
| 0.2 | 0.1 | 138 |
| 6.1 | 0.1 | 130 |
| 0.05 | 0.05 | 132 |

1) Non-ionic detergent inhibition is not significant at low concentrations.
2) An ionic detergent inhibitor at high concentrations.
3) Mixture of anionic and non-ionic detergents stimulate activity.

STABILITY TOWARD OXIDANTS

Cutinase .5 mg/ml in 0.1m sodium phosphate buffer, was incubated with various levels of hydrogen peroxide at pH 8.4 and 25°C for 2 hours, and hydrolytic activity was measured by a pH-stat using trioctanoim-polyvinyl alcohol emulsion.

| [$H_2O_2$ ppm] | Hydrolytic Activity %<br>Remaining |
|---|---|
| 0 | 100 |
| 100 | 86 |
| 200 | 86 |
| 500 | 91 |
| 1000 | 95 |

TABLE 1

pH-optimum of bacterial cutinase.

50 mM NaP, 30 C, 0.5 % TO.

Act. (mmol/mg.min)

pH

☐ Hydrolase activity.

TABLE 2

Temperature optimum of bact. cutinase.
50 mM NaP, pH 8.0.

Act. (mmol/mg·min)

Temp. (C)

□ Hydrolase activity.

**Claims**

1. An enzymatic cleaning composition comprising a surfactant, a substantially pure microbial cutinase and one or more enzymes selected from lipases, amylases and proteases.

2. A composition according to claim 1 wherein the cutinase is present in an amount of from about 0.01% to about 5% by weight of the surfactant.

3. A composition according to claim 1 or claim 2 wherein the cutinase is that isolable from ATCC 53552.

4. A composition according to any one of claims 1 to 3 wherein the cutinase is a non-naturally occurring cutinase having at least one amino acid randomly or selectively replaced by an amino acid not naturally found at that position.

5. A composition according to any one of claims 1 to 4 wherein the cutinase is added in the form of cell fermentation broth containing cutinase but essentially free of other enzymes or enzyme sources.

6. A composition according to claim 1 comprising a plurality of surfactants.

7. A composition according to claim 6 wherein the surfactants are sodium dodecylsulphate (SDS) and Triton X-100.

8. An enzymatic cleaning additive comprising a substantially pure microbially produced cutinase and one or more enzymes selected from lipases, amylases or proteases.

9. A method for enzymatically cleaning a material having a cutin or cutin-like stain comprising:
   a) selecting a cutinase enzyme and one or more enzymes selected from lipases, amylases or proteases;
   b) forming an aqueous solution of the enzymes with said cutinase enzyme in a concentration of from about 0.05mg/l to 100mg/l;
   c) contacting the material with the solution of step b); and
   d) rinsing the material of step c).

10. A method according to claim 9 wherein the aqueous solution further comprises a surfactant compatible with the selected cutinase.

11. A method according to claim 9 wherein the selected cutinase causes at least 10% hydrolysis of the cutin or cutin-like stain.

12. A method of manufacture of an enzymatic cleaning composition comprising adding a substantially pure microbial cutinase and one or more enzymes selected from lipases, amylases or proteases to a surfactant.

**Patentansprüche**

1. Enzymatische Reinigungsmittelzusammensetzung umfassend eine oberflächenaktive Substanz, eine im wesentlichen reine mikrobielle Cutinase und ein oder mehrere Enzyme ausgewählt aus Lipasen, Amylasen und Proteasen.

2. Zusammensetzung nach Anspruch 1, worin die Cutinase in einer Menge von etwa 0,01 Gew.-% bis etwa 5 Gew.-% der oberflächenaktiven Substanz vorhanden ist.

3. Zusammensetzung nach Anspruch 1 oder Anspruch 2, worin die Cutinase jene ist, die aus ATCC 53552 isolierbar ist.

4. Zusammensetzung nach einem der Ansprüche 1 bis 3, worin die Cutinase eine nicht natürlich vorkommende Cutinase ist, in der zumindest eine Aminosäure zufällig oder selektiv durch eine Aminosäure ersetzt ist, die in der Natur an dieser Position nicht vorkommt.

5. Zusammensetzung nach einem der Ansprüche 1 bis 4, worin die Cutinase in Form einer Zellfermentationsbrühe zugesetzt wird, die Cutinase enthält, aber im wesentlichen frei von anderen Enzymen oder Enzymquellen ist.

6. Zusammensetzung nach Anspruch 1 umfassend eine Vielzahl an oberflächenaktiven Substanzen.

7. Zusammensetzung nach Anspruch 6, worin die oberflächenaktiven Substanzen Natriumdodecylsulfat (SDS) und Triton X-100 sind.

8. Enzymatisches Reinigungsmitteladditiv umfassend eine im wesentlichen reine mikrobiell gebildete Cutinase und ein oder mehrere Enzyme ausgewählt aus Lipasen, Amylasen oder Proteasen.

9. Verfahren zum enzymatischen Reinigen eines eine Cutin- oder Cutin-artige Verfärbung aufweisenden Materials, umfassend:
   a) das Auswählen eines Cutinaseenzyms und eines oder mehrerer aus Lipasen, Amylasen oder Proteasen ausgewählter Enzyme;
   b) das Bilden einer wässerigen Lösung der Enzyme, wobei das genannte Cutinaseenzym in einer Konzentration von etwa 0,05 mg/l bis 100 mg/l vorliegt;
   c) das In-Kontakt-Bringen des Materials mit der Lösung von Schritt b); und
   d) das Spülen des Materials von Schritt c).

10. Verfahren nach Anspruch 9, worin die wässerige Lösung weiters eine mit der ausgewählten Cutinase kompatible oberflächenaktive Substanz umfaßt.

11. Verfahren nach Anspruch 9, worin die ausgewählte Cutinase eine zumindest 10%ige Hydrolyse der Cutin- oder Cutin-artigen Verfärbung bewirkt.

12. Verfahren zur Herstellung einer enzymatischen Reinigungsmittelzusammensetzung, umfassend das Hinzufügen einer im wesentlichen reinen mikrobiellen Cutinase und eines oder mehrerer aus Lipasen, Amylasen oder Proteasen ausgewählter Enzyme zu einer oberflächenaktiven Substanz.

## Revendications

1. Composition enzymatique de nettoyage comprenant un agent tensio-actif, une cutinase microbienne sensiblement pure et une ou plusieurs enzymes choisies parmi les lipases, les amylases et les protéases.

2. Composition selon la revendication 1, où la cutinase est présente en une quantité comprise entre environ 0,01% et environ 5% en poids de l'agent tensio-actif.

3. Composition selon la revendication 1 ou la revendication 2, où la cutinase est celle pouvant être isolée de ATCC 53552.

4. Composition selon l'une quelconque des revendications 1 à 3, où la cutinase est une cutinase ne se présentant pas naturellement, ayant au moins un acide aminé remplacé statistiquement ou sélectivement par un acide aminé que l'on ne trouve pas naturellement en cette position.

5. Composition selon l'une quelconque des revendications 1 à 4, où la cutinase est ajoutée sous la forme d'un bouillon de fermentation de cellules contenant de la cutinase mais essentiellement exempt d'autres enzymes ou sources d'enzyme.

6. Composition selon la revendication 1, comprenant un certain nombre d'agents tensio-actifs.

7. Composition selon la revendication 6, où les agents tensio-actifs sont le dodécylsulphate de sodium (SDS) et Triton X-100.

8. Additif enzymatique de nettoyage comprenant une cutinase microbiennement produite sensiblement pure et une ou plusieurs enzymes choisies parmi les lipases, les amylases ou les protéases.

9. Méthode pour le nettoyage enzymatique d'une matière ayant une tache de cutine ou ressemblant à la cutine, comprenant :
   a) la sélection d'une enzyme de cutinase et d'une ou plusieurs enzymes choisies parmi les lipases, les amylases ou les protéases;

b) la formation d'une solution aqueuse des enzymes avec ladite enzyme de cutinase à une concentration comprise entre environ 0,05mg/l et 100mg/l;

c) la mise en contact de la matière avec la solution de l'étape b); et

d) le rinçage de la matière de l'étape c).

10. Méthode selon la revendication 9, où la solution aqueuse contient de plus un agent tensio-actif compatible avec la cutinase choisie.

11. Méthode selon la revendication 9, où la cutinase choisie provoque une hydrolyse à au moins 10% de la tâche de cutine ou ressemblant à la cutine.

12. Méthode de fabrication d'une composition enzymatique de nettoyage consistant à ajouter une cutinase microbienne sensiblement pure et une ou plusieurs enzymes choisies parmi les lipases, amylases ou protéases, à un agent tensio-actif.